# EUROPÄISCHE PATENTSCHRIFT

(11) **EP 1 753 485 B1**
(45) Veröffentlichungstag und Bekanntmachung des Hinweises auf die Patenterteilung: **01.08.2012**
(21) Anmeldenummer: 05753775.5
(22) Anmeldetag: 03.06.2005
(51) Int. Cl.: A61M 1/28, A61M 39/20, A61J 1/05

(54) **BEUTELSET FÜR DIE PERITONEALDIALYSE UND DESSEN VERWENDUNG**
BAG SET FOR PERITONEAL DIALYSIS AND USE THEREOF
ENSEMBLE DE SACS POUR LA DIALYSE PERITONEALE ET SON UTILISATION

(30) Priorität: 07.06.2004 DE 102004027743
(43) Veröffentlichungstag der Anmeldung: 21.02.2007
(73) Patentinhaber: Fresenius Medical Care Deutschland GmbH, 66606 Strasse Wendel (DE)
(72) Erfinder: BIESEL, Wolfgang, 66564 Ottweiler (DE)
(74) Vertreter: Vièl, Christof
(86) Internationale Anmeldenummer: PCT/DE2005/001002
(87) Internationale Veröffentlichungsnummer: WO 2005/120604

(56) Entgegenhaltungen:
- WO-A-86/02907
- WO-A-94/28855
- DE-A1- 10 042 067

## Beschreibung

Patientenkonnektoren für die Peritonealdialyse sind beispielsweise aus der EP 0 715 860 B1 oder der DE 198 14 047 C1 bekannt. Diese ermöglichen eine sterile Konnektion und Dekonnektion im Rahmen einer Peritonealdialyse-Behandlung. Nach Beendigung der Peritonealdialyse wird mit derartigen Patientenkonnektoren ein Verschlußstopfen in den Konnektor des Schlauchstückes, das in die Bauchhöhle des Patienten geht, eingebracht. Darüber hinaus wird in der Regel eine sterile Desinfektionskappe auf den Bauchanschluß aufgebracht, um das Eindringen von Keimen in diesen zu verhindern. Somit ist der Zugang zur Bauchhöhle des Patienten steril verschlossen.

Zwar ist jeweils auf dem üblicherweise als Beutel ausgebildeten Behälter angegeben, welche Flüssigkeit in welcher Menge darin enthalten ist; jedoch liegen diese Informationen nach Einfüllen der Flüssigkeit und Beseitigung des Beutels nicht mehr vor. Da beispielsweise bei der CAPD (continous ambulatory peritoneal dialysis) Lösungen mit unterschiedlichen Glukosekonzentrationen, osmotischen Komponenten oder Nährstoffen in einer bestimmten Abfolge in einem bestimmten Zeitraum vom Patienten verwendet werden müssen, kann es leicht dazu kommen, daß dieser nicht mehr weiß, welche Flüssigkeit derzeit im Bauchraum vorliegt und in welcher Menge. Noch problematischer sind Fälle, bei denen der Patient bewußtlos wird und keine Angaben mehr zu der zuletzt eingefüllten Lösung machen kann oder aber bei einer Krankenhausbehandlung mit schichtweise wechselndem Personal, wo es zu Lücken in der Notierung kommen kann.

Aus der WO94/28855 A1 ist ein Anschtußschutz zum Aufrechterhalten einer sterilen Umbebung im Innern eines rohrförmigen Elementes wie etwa eines Anschlusses bekannt. Die WO86/02907 A1 beschreibt einen Verschluß für einen Behälter für medizinische Lösungen.

In Anbetracht der teilweise gravierenden Konsequenzen einer falschen Flüssigkeitsabfolge im Bauchraum liegt der Erfindung die Aufgabe zugrunde, eine falsche Flüssigkeitsabfolge bei der Peritonealdialyse zu verhindern.

Dies wird erfindungsgemäß den Kennzeichnenden Teil von Anspruch 1 durch gelöst,

Auf diese Weise ist es auch nach der Befüllung des Bauchraumes und Beseitigen des Beutels jederzeit möglich, das derzeit im Bauchraum befindliche Medium zu ermitteln bzw. andere Angaben, wie Menge, Konzentration, Hersteller, etc. des Mediums zu erfassen und dies insbesondere bei nachfolgenden Schritten der Peritionealdialyse zu berücksichtigen.

Es ist im Rahmen der Erfindung vorgesehen, daß die Informationen die Art des Mediums betreffen.

Diese Informationen können sowohl die jeweils verwendete Lösung betreffen, z.B. zur Unterscheidung einer glukosehaltigen von einer aminosäurehaltigen Lösung dienen.

Ebenfalls ist vorgesehen, daß die Informationen die Menge des Mediums betreffen.

Weiterhin ist es zweckmäßig, daß die Informationen die Konzentration des Mediums betreffen.

Da in der Peritonealdialyse Lösungen mit unterschiedlicher Konzentration eines Mediums verwendet werden, z.B. Calciumlösungen mit 1,0 %, mit 1,25 % oder mit 1,75 % Calcium, ist es sinnvoll, auch die Konzentration in die Kodierung mit einzubeziehen.

Es ist zur Erfindung gehörig, daß die Informationen in tastbarer Form in Braille ausgebildet sind.

Weiterhin ist es erfindungsgemäß, daß die Kappe eine Desinfektions kappe sein kann.

Imn Rahmen der Erfindung lieft auch ein Verfahren zur Verwendung eines Beutelsets für die Peritonealdialyse, enthaltend mindestens einen Beutel mit einem Medium und eine Kappe, die zum Verschließen und Schützen vor Verkeimung des Bauchanschlusses des Patienten geeignet ist, wobei die Kappe Informationen über das Medium in Form eines Farbcodes, eines Barcodes von Symbolen oder in tastbarer Form aufweist, wobei nach Befüllen des Bauchraums des Patienten der Bauchanschluß durch die Kappe verschlossen wird, so daß die im Peritoneum enthaltene Flüssigkeit an der jeweils verwendeten Kappe abgelesen werden kann.

Im diesem Zusammenhang ist vorteilhaft daß in einer Detektionsvorrichtung, die Mittel aufweist, um an einer Kappe vorgesehene Informationen über das jeweils im Peritoneum enthaltene Medium zu verarbeiten, eine Abfolge der zu verwendenden Medien gespeichert wird und die Detektionsvorrichtung beim Anschließen eines unzutreffenden Mediums einen Alarm auslöst.

Es ist hierbei möglich, daß die Informationen die Art des Mediums betreffen.

Ebenfalls ist es möglich, daß die Informationen die Menge des Mediums betreffen.

Es kann auch zwechmäßig sein, daß die Informationen die Konzentration des Mediums betreffen.

Es ist vorteilhaft, daß die tastbaren Informationen in Braille ausgebildet sind.

Schließlich ist es sinnvoll, daß die Kappe (4) eine Desinfektionskappe ist.

Auf diese Weise ist sichergestellt, daß mit jedem Beutelsystem die für es charakteristische Kappe mitgeliefert wird, so daß zum einen immer eine neue, sterile Kappe verwendet wird und zum anderen das Beutelsystem durch die mit der Information versehene Kappe sichtbar gekennzeichnet ist.

Die Detektionsvorrichtung kann zum Ausgeben der Informationen dienen, sei es durch eine Text- oder Sprachanzeige, so daß neben dem Benutzer auch Dritte (z.B. das Betreuungspersonal in einem Krankenhaus) die Informationen zu der derzeit verwendeten Lösung erhalten können.

Die Vorteile der Erfindung bestehen darin, daß nicht nur der Benutzer, sondern auch Dritte, gegebenenfalls mit Hilfe einer Detektionsvorrichtung, die relevanten Informationen zu der jeweils bei der Peritonealdialyse verwendeten Lösung in Erfahrung bringen können und daß zudem bei einer falschen Abfolge der Lösungen automatisch eine Warnung ausgelöst werden kann.

Nachfolgend wird ein Ausführungsbeispiel der Erfindung anhand einer Zeichnung erläutert.

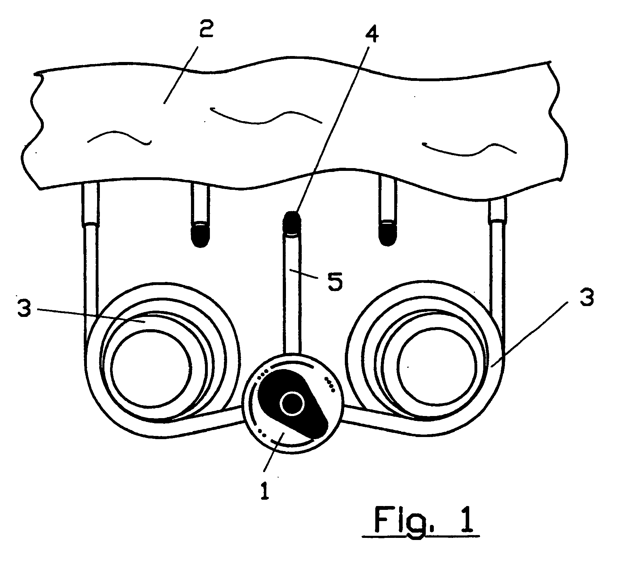

Es zeigt
- Fig. 1: einen Patientenkonnektor mit einer erfindungsgemäßen Kappe.

Wie aus Fig. 1 ersichtlich, ist ein Patientenkonnektor 1 mit einem Beutelsystem 2 für die Peritonealdialyse über Schläuche 3 verbunden. Der Patientenkonnektor 1 weist eine Kappe 4, insbesondere eine Desinfektionskappe 4 auf, die über ein Zwischenstück 5 mit dem Patientenkonnektor 1 verbunden ist. Das Zwischenstück 5 ist in der Mitte geteilt und hält sowohl die Kappe 4 als auch den Patientenkonnektor 1 in einem sterilen Zustand. Der Patientenkonnektor 1 wird mit dem Bauchanschluß des Patienten verbunden, um einen Wechsel der Lösungen vorzunehmen. Anschließend wird der Bauchanschluß wieder durch einen Verschlußstopfen und die Kappe 4 oder durch eine Kappe 4, die auch die Funktion des Verschließens übernimmt, verschlossen.

Um Verwechslungen zu vermeiden und die Informationen zu der jeweils verwendeten Lösung jeweils abrufen zu können, wird eine Kappe 4 verwendet, die diese Informationen beispielsweise durch einen Farbcode, Barcode, in Form von Symbolen oder in einer tastbaren Kodierung (z.B. in Braille) aufweist. Diese Informationen können mit einer entsprechenden Detektionsvorrichtung gelesen werden, welche über eine Farberkennung, einen Barcodeleser oder einen optischen Sensor verfügt.

Ein Beispiel für eine derartige Kodierung ist in folgender Liste wiedergegeben:

| **Stoff/Konzentration** | **Farbe** | **tastbare Erhebung** |
|---|---|---|
| 1,5 % Glukose | gelb | 1 |
| 2,3 % Glukose | grün | 2 |
| 4,25 % Glukose | rot | 3 |
| Aminosäure | blau | 4 |
| Polyglukose | violett | 5 |
| 1,0 % Calcium (Ca⁺⁺) | 0-33 % schwarz | keine |
| 1,25 % Calcium (Ca⁺⁺) | 34-66 % schwarz | keine |
| 1,75 % Calcium (Ca⁺⁺) | 67-100 % schwarz | keine |

Für weitere Stoffe, die in Dialyselösungen Verwendung finden, kann selbstverständlich eine entsprechende Kodierung vorgesehen werden. Ebenfalls ist es möglich, Kodierkombinationen vorzusehen, z.B. für eine bestimmte Glukosekonzentration und eine bestimmte Calciumkonzentration.

Die Kappe 4 verbleibt nach dem Befüllen des Peritoneums auf dem Bauchanschluß des Patienten, so daß die im Peritoneum enthaltene Flüssigkeit an der jeweils verwendeten Desinfektionskappe 4 abgelesen werden kann.

Auf diese Weise ist es auch möglich, auch bei Bewußtlosigkeit des Patienten die Informationen abzurufen bzw. bei einer unrichtigen Abfolge der Lösungen einen Warnhinweis zu erhalten.

## Patentansprüche

1. Beutelset für die Peritonealdialyse, enthaltend mindestens einen Beutel (2) mit einem Medium und eine Kappe (4), wobei die Kappe (4) Informationen über das Medium in Form eines Farbcodes, eines Barcodes, von Symbolen oder in tastbarer Form aufweist, **dadurch gekennzeichnet, daß** die Kappe (4) zum Verschließen und Schützen vor Verkeimung des Bauchanschlusses des Patienten geeignet ist,

2. Beutelset gemäß Anspruch 1, **dadurch gekennzeichnet, daß** die Informationen die Art des Mediums betreffen.

3. Beutelset gemäß Anspruch 1, **dadurch gekennzeichnet, daß** die Informationen die Menge des Mediums betreffen.

4. Beutelset gemäß Anspruch 1, **dadurch gekennzeichnet, daß** die Informationen die Konzentration des Mediums betreffen.

5. Beutelset gemäß Anspruch 1, **dadurch gekennzeichnet, daß** die tastbaren Informationen in Braille ausgebildet sind.

6. Beutelset gemäß Anspruch 1, **dadurch gekennzeichnet, daß** die Kappe (4) eine Desinfektionskappe ist.

7. Verfahren zur Verwendung eines Beutelsets für die Peritonealdialyse, enthaltend mindestens einen Beutel (2) mit einem Medium und eine Kappe (4), die zum Verschließen und Schützen vor Verkeimung des Bauchanschlusses des Patienten geeignet ist, wobei die Kappe (4) Informationen über das Medium in Form eines Farbcodes, eines Barcodes von Symbolen oder in tastbarer Form aufweist, wobei nach Befüllen des Bauchraums des Patienten der Bauchanschluß durch die Kappe (4) verschlossen wird, so daß die im Peritoneum enthaltene Flüssigkeit an der jeweils verwendeten Kappe (4) abgelesen werden kann.

8. Verfahren gemäß Anspruch 7, dadurch gekennzeiclmet, daß in einer Detektionsvorrichtung, die Mittel aufweist, um an einer Kappe vorgesehene Informationen über das jeweils im Peritoneum enthaltene Medium zu verarbeiten, eine Abfolge der zu verwendenden Medien gespeichert wird und die Detektionsvorrichtung beim Anschließen eines unzutreffenden Mediums einen Alarm auslöst.

9. Verfahren gemäß Anspruch 7, **dadurch gekennzeichnet, daß** die Informationen die Art des Mediums betreffen.

10. Verfahren gemäß Anspruch 7, **dadurch gekennzeichnet, daß** die Informationen die Menge des Mediums betreffen.

11. Verfahren gemäß Anspruch 7, **dadurch gekennzeichnet, daß** die Informationen die Konzentration des Mediums betreffen.

12. Verfahren gemäß Anspruch 7, **dadurch gekennzeichnet, daß** die tastbaren Informationen in Braille ausgebildet sind.

13. Verfahren gemäß Anspruch 7, **dadurch gekennzeichnet, daß** die Kappe (4) eine Desinfektionskappe ist.

## Claims

1. Bag set for peritoneal dialysis, containing at least one bag (2) with a medium and a cap (4), the cap (4) having information concerning the medium in the form of a colour code, a bar code, symbols or in touch-type form, **characterised in that** the cap (4) is suitable for closing off the patient's abdominal connector and protecting it from germs.

2. Bag set according to claim 1, **characterised in that** the information concerns the kind of medium.

3. Bag set according to claim 1, **characterised in that** the information concerns the amount of medium.

4. Bag set according to claim 1, **characterised in that** the information concerns the medium's concentration.

5. Bag set according to claim 1, **characterised in that** the touch-type information is in the form of Braille.

6. Bag set according to claim 1, **characterised in that** the cap (4) is a disinfectant cap.

7. Method of using a bag set for peritoneal dialysis, containing at least one bag (2) with a medium and a cap (4) which is suitable for closing off the patient's abdominal connector and protecting it from germs, the cap (4) having information concerning the medium in the form of a colour code, a bar code, symbols or in touch-type form, the abdominal connector being closed off by means of the cap (4) after the patient's abdominal cavity has been filled so that the fluid contained in the peritoneum can be read off from the cap (4) used in each case.

8. Method according to claim 7, **characterised in that** a sequence of the media to be used is stored in a detection device that has means for processing information provided on a cap and relating to the medium contained at any one time in the peritoneum, and that the detection device triggers an alarm if an unsuitable medium is connected up.

9. Method according to claim 7, **characterised in that** the information concerns the kind of medium.

10. Method according to claim 7, **characterised in that** the information concerns the amount of medium.

11. Method according to claim 7, **characterised in that** the information concerns the medium's concentration.

12. Method according to claim 7, **characterised in that** the touch-type information is in the form of Braille.

13. Method according to claim 7, **characterised in that** the cap (4) is a disinfectant cap.

## Revendications

1. Ensemble de poches pour la dialyse péritonéale, comprenant au moins une poche (2) avec un fluide et un capuchon (4), le capuchon (4) présentant des informations sur le fluide sous la forme d'un code de couleur, d'un code barres, de symboles ou sous une forme tactile, **caractérisé en ce que** le capuchon (4) est apte à fermer et à protéger de la contamination microbienne le raccord abdominal du patient.

2. Ensemble de poches selon la revendication 1, **caractérisé en ce que** les informations concernent la nature du fluide.

3. Ensemble de poches selon la revendication 1, **caractérisé en ce que** les informations concernent la quantité du fluide.

4. Ensemble de poches selon la revendication 1, **caractérisé en ce que** les informations concernent la concentration du fluide.

5. Ensemble de poches selon la revendication 1, **caractérisé en ce que** les informations tactiles sont formées en Braille.

6. Ensemble de poches selon la revendication 1, **caractérisé en ce que** le capuchon (4) est un capuchon désinfectant.

7. Procédé pour l'utilisation d'un ensemble de poches pour la dialyse péritonéale, comprenant au moins une poche (2) avec un fluide et un capuchon (4) apte à fermer et à protéger de la contamination microbienne le raccord abdominal du patient, le capuchon (4) comprenant des informations sur le fluide sous la forme d'un code de couleur, d'un code barres, de symboles ou sous une forme tactile, le raccord abdominal étant fermé par le capuchon (4) après remplissage de la cavité péritonéale du patient, de sorte qu'il est possible d'identifier le liquide contenu dans le péritoine à l'aide du capuchon (4) utilisé.

8. Procédé selon la revendication 7, **caractérisé en ce qu'**une suite des fluides à utiliser est mémorisée dans un dispositif de détection comprenant des moyens pour traiter des informations prévues sur un capuchon concernant le fluide contenu dans le péritoine, et le dispositif de détection déclenche une alarme en cas de raccordement d'un fluide inapproprié.

9. Procédé selon la revendication 7, **caractérisé en ce que** les informations concernent la nature du fluide.

10. Procédé selon la revendication 7, **caractérisé en ce que** les informations concernent la quantité du fluide.

11. Procédé selon la revendication 7, **caractérisé en ce que** les informations concernent la concentration du fluide.

12. Procédé selon la revendication 7, **caractérisé en ce que** les informations tactiles sont formées en Braille.

13. Procédé selon la revendication 7, **caractérisé en ce que** le capuchon (4) est un capuchon désinfectant.
